# EUROPEAN PATENT APPLICATION

(11) **EP 4 541 324 A1**
(43) Date of publication of application: **23.04.2025**
(21) Application number: 23824256.4
(22) Date of filing: 15.06.2023
(51) Int. Cl.: A61F 2/44, A61F 2/30

(54) **ARTIFICIAL LUMBAR DISC**

(30) Priority: 16.06.2022 KR 20220073251
(71) Applicant: HUB Biotech Co., Ltd, Seoul 08506 (KR); Yoon, Kang Jun, Seoul 06274 (KR)
(72) Inventor: YOON, Kang Jun, Seoul 06274 (KR)
(74) Representative: Bianchetti & Minoja with Trevisan & Cuonzo IPS SRL
(86) International application number: PCT/KR2023/008307
(87) International publication number: WO 2023/244043

(57) **Abstract**

The present disclosure relates to an artificial lumbar disc and provides a lateral approach artificial disc that is inserted laterally rather than anteriorly of the spine, thereby preventing damage to blood vessels or other organs that may occur when an anterior access artificial disc is inserted from the front, shortening the surgical time, preventing dislodgment of the artificial disc, and improving the surgical prognosis. The artificial disc is inserted between two adjacent vertebrae and includes an upper plate contacting an upper vertebra, a lower plate contacting a lower vertebra, and an inserter inserted between the upper plate and the lower plate to allow the upper plate to rotate by a set angle with respect to the lower plate, and an upper wing portion and a lower wing portion are formed on the upper plate and the lower plate, which extend in an insertion direction and an installer direction in a state in which the inserter is inserted.

## Description

### Technical Field

The present disclosure relates to an artificial lumbar disc, and more particularly, to an artificial lumbar disc that may be inserted from a lateral approach into a vertebral body.

### Background Art

The vertebral column consists of 32 to 35 vertebrae, which make up the torso, and intervertebral disks, or discs, between the vertebrae, and it is the central portion of the body, connecting the skull at the top and the pelvis at the bottom.

The vertebrae include 7 cervical vertebrae, 12 thoracic vertebrae, 5 lumbar vertebrae, 5 sacrum vertebrae, and 3 to 5 coccyx vertebrae from the top. In the case of adults, 5 sacral vertebrae fuse to form 1 sacrum, and 3 to 5 coccyx vertebrae fuse to form 1 coccyx.

Between the vertebrae, there is a cartilaginous substance called a disc. The cartilaginous substance is a soft and pliable fibrocartilage that forms cartilaginous joints, acts like a ligament that helps the spine move flexibly and stabilizes the spine, and absorbs and reduces shocks applied to the vertebral body. The intervertebral disc is composed of a fibrous disc with a fibrous outer portion and a nucleus with a gel-like substance inside, which helps to maintain strength and disperse stress. In other words, the intervertebral disc absorbs the body's load and shocks between each vertebra except for some of the cervical vertebrae, acts as a buffer that disperses shocks like a spring, holds the vertebrae so that they do not move away, securely provides a smooth range of spinal joint space by separating two vertebrae so that the spinal nerves are not compressed, and plays a role in facilitating the movement of each vertebra.

Because the intervertebral discs buffer or transmit a load and shocks applied in a vertical direction, various diseases may occur, such as spinal stenosis, osteophyte formation, disc herniation, and nerve root compression.

As one of treatment methods for serious spinal diseases, a spinal fusion has been used for a long time. The spinal fusion is a surgical method in which the intervertebral disc is removed and a cage to replace the intervertebral disc is inserted to fuse adjacent vertebrae.

When the spinal fusion is performed on the lumbar spine, the spinal fusion may be categorized into posterial lumbar interbody fusion (PLIF), transformational lumbar interbody fusion (TLIF), lateral lumbar interbody fusion (LLIF), oblique lumbar interbody fusion (OLIF), and anterior lumbar interbody fusion (ALIF) depending on the direction of cage insertion.

In PLIF, an incision is made along the midline of the spine, the incision portion is opened to expose the entire vertebral body, a portion of the posterior part of the vertebra is removed, the disc is removed, and a PLIF cage is inserted.

PLIF is the oldest spinal fusion technique and is an essential method when performing two- or three-joint fusions. However, there is a high possibility of adhesion to nerves, ligaments, and muscles due to the surgical procedure, the healing time is long because the incision area is large, and some people have significant aftereffects.

The PLIF cage includes a pair of small cages placed on both sides of the spine, and is the smallest cage used in all spinal fusions.

TLIF is a surgical method in which a small incision is made along both sides of spinal muscles, the vertebral body is minimally exposed, and a TLIF cage is inserted while removing the spinal joint area in a direction of the neural foramen. This surgical technique has the advantage of reducing bleeding and shortening the surgical time, so it is suitable for single-joint surgeries, but PLIF surgery is required when multiple areas require surgery. The TLIF cages are mostly arc-shaped, and thus, are inserted into the vertebral column and rotated so that the convex part of the TLIF cage faces the ventral side. TLIF cages are larger than the PLIF cages, but their support area is smaller than that of the LLIF cage or ALIF cage, which will be mentioned later.

ALIF has many advantages, such as quick recovery from surgery and no need to worry about adhesions, but it has the disadvantage of requiring highly skilled techniques because it is performed by making an incision in the front ventral side to remove internal organs and approaching the spine. The ALIF cages have the advantage of having the largest support area among all spinal fusion cages.

LLIF was developed to overcome the shortcomings of ALIF, PLIF, and TLIF. Because LLIF is performed through an incision in a side, compared to existing surgeries that involve back incision, the LLIF method may widen a space of stenotic areas between the vertebrae and has the advantage of causing little damage to the surrounding tissue. However, because there are psoas muscle and peritoneum around the surgical path, there are problems such as thigh muscle paralysis if there is a mistake during the surgery. The LLIF cage is smaller than the ALIF cage, but larger than the PLIF or TLIF cage.

Compared to the LLIF, OLIF or Anterior To Psoas (ATP) fusion is a safer and more effective surgical method. OLIF has the advantage of allowing a surgical path to be made in an inclined direction from a side, and may be performed between the 4th lumbar L4 and 5th lumbar L5 vertebrae, which are difficult to operate on with DLIF due to the psoas muscle and peritoneum. In addition, the possibility of damaging nerves that may be problem in LLIF is significantly lower.

However, because the above-mentioned spinal fusion makes the upper and lower vertebrae into one, and thus, from the patient's perspective, one of the corresponding discs is missing, the degree of mobility that the disc was responsible for is lost.

Artificial discs have been developed to solve the problem of loss of mobility. In a surgical method using artificial discs, first, the damaged intervertebral disc is removed, a space is created between two adjacent vertebrae, and then an artificial disc is inserted into the space. Artificial discs have the advantage of maintaining mobility even after surgery because the upper and lower plates may move within a certain range of angles.

In conventional artificial disc replacement surgery, because the posterior arch, which consists of a pair of pedicles, the superior and inferior articular processes extending posteriorly from the pedicles, lamina propria, and supraspinatus, is located on the dorsal, after the abdomen is opened, the peritoneum is removed, and the organs are moved, the surgery is performed from the anterior approach.

In more detail, in a state wherein, first, the patient is laid down with the front facing upward, the abdomen on the front of the body is incised and a surgical path is secured to the spine, avoiding the organs. Then, a portion of an anterior longitudinal ligament is incised in the front side of the spine. When the anterior longitudinal ligament is incised, the disc between the vertebrae is exposed. The disc is removed using a surgical instrument, and an artificial disc is inserted in the place of the disc.

Therefore, the conventional surgical method using an artificial disc has the problem that the incision area is large, the surgery must be performed while avoiding organs, so there is a possibility of damaging the surrounding blood vessels and tissues, and the surgical time increases. In particular, since the anterior longitudinal ligament is incised, there is a bigger problem that the patient's postoperative prognosis is not good.

Another problem due to the incision of the anterior longitudinal ligament is that, although the original human disc is blocked by the anterior longitudinal ligament, if an artificial disc is inserted with the anterior longitudinal ligament incised, when a load is applied in a direction of the long axis of the human body, the force is transmitted forward, which may cause the artificial disc to fall out in a forward direction.

In addition, there is a problem in that an excessive force is transmitted to the posterior joint due to the removal of the anterior longitudinal ligament, which may cause another spinal deformation.

### Disclosure of Invention

### Technical Problem

The present disclosure is intended to solve the above problems and provides a lateral approach artificial disc that is inserted laterally rather than anteriorly of the spine, thereby preventing damage to blood vessels or other organs that may occur when an anterior access artificial disc is inserted from the front, shortening the surgical time, preventing dislocation of the artificial disc, and improving the surgical prognosis.

### Solution to Problem

In an artificial disc inserted between two adjacent vertebrae, according to the present disclosure, the artificial disc includes an upper plate contacting upper vertebrae, a lower plate contacting lower vertebrae, and an inserter inserted between the upper plate and the lower plate to allow the upper plate to rotate at a set angle with respect to the lower plate, wherein the upper plate and the lower plate are characterized in that an upper wing portion and a lower wing portion are formed along a lateral axis while the inserter is inserted.

The upper plate and the lower plate may have a thickness greater in an anterior direction than in a posterior direction.

An upper keel portion and a lower keel portion may be formed along a lateral axis on an upper plate outer surface of the upper plate and a lower plate outer surface of the lower plate, respectively.

A plurality of upper protrusions and lower protrusions may be formed on an upper plate outer surface of the upper plate and a lower plate outer surface of the lower plate, respectively.

The plurality of upper protrusions and the lower protrusions may have a quadrangular pyramid shape with a quadrangular cross section composed of four line segments each perpendicular to the lateral axis and the anterior to posterior axis.

The inserter may include an upper dome portion on an upper side thereof and a first axial rotation portion on a lower side thereof, an upper groove corresponding to the upper dome portion may be formed on the upper plate and may be in sliding contact with the upper dome portion, and a second axial rotation portion may be formed on the lower plate that contacts the first axial rotation portion.

The upper dome portion may be formed in an elliptical hemispherical shape in which a lateral axis is arranged on the lateral axis line and a minor axis is arranged on the anterior to posterior axis line, and the upper plate may perform lateral bending and anterior to posterior flexion-extension motion with respect to the lower plate by moving the upper dome portion and upper groove in contacting each other.

A maximum depth of the upper groove may be formed less than a maximum height of the upper dome portion.

An upper stopper may be formed around the upper groove, and an angular momentum of the upper plate may be limited by the upper stopper coming into contact with an inserter upper surface of the inserter body formed around the upper dome portion.

An inserter lower surface of the inserter body may be formed flat, and the inserter upper surface may be formed so that a thickness thereof is gradually reduced from the upper dome portion to an end of the inserter body.

The first axial rotation portion may be a lower outer protrusion formed on the inserter lower surface, and the second axial rotation portion may be a lower outer groove formed on the lower plate, and the above lower outer groove may be formed in an arc shape, wherein the lower outer groove may be formed in an arc shape.

A lower center protrusion may be formed at the center of the inserter lower surface, and a lower center groove corresponding to the lower center protrusion may be formed on the lower plate.

### Advantageous Effects of Invention

According to an embodiment of the present disclosure configured as described above, an artificial lumbar disc that allows insertion laterally of a spine, and thus, may prevent damage to blood vessels or other organs during a surgery and shorten the surgery time.

In addition, if such an artificial disc is inserted, the anterior longitudinal ligament is not removed, and thus, there is an advantage in that the dislocation of the artificial disc after surgery may be prevented and the function of the anterior longitudinal ligament may be maintained as it is.

In addition, because the artificial lumbar disc is inserted laterally, it has the advantage of not only preventing damage to organs, but also reducing the surgery time, so an amount of bleeding during the patient's surgery may be suppressed.

### Brief Description of Drawings

FIG. 1 is a perspective view of an artificial disc according to an embodiment of the present disclosure.
FIG. 2 is a side view of the artificial disc of FIG. 1 viewed from an insertion direction.
FIG. 3 is an exploded perspective view of the artificial disc of FIG. 1 viewed from an upper side.
FIG. 4 is an exploded perspective view of the artificial disc of FIG. 1 viewed from a lower side.
FIG. 5 is a view of an inserter of the artificial disc of Figure 1 viewed from the insertion direction.
FIG. 6 is a view of the inserter of the artificial disc of FIG. 1 viewed from an anterior direction.
FIG. 7 is a diagram for explaining implementation of a lateral bending and anterior to posterior flexion-extension motion of the inserter of the artificial disc of FIG. 1.
FIG. 8 is a diagram for explaining implementation of axial rotating of the inserter of the artificial disc of FIG. 1.
FIG. 9 is a diagram showing insertion of the artificial disc of FIG. 1 into the vertebral body.
FIG. 10 is a diagram showing the artificial disc of FIG. 1 inserted into the vertebral body.

### Best Mode for Carrying out the Invention

Hereinafter, embodiments of the present disclosure will be described in detail with reference to the accompanying drawings. When assigning reference numerals to components of each drawing below, identical components are given the same reference numerals as much as possible even if they are shown in different drawings, and detailed descriptions of known functions and configurations that may unnecessarily make unclear the gist of the present disclosure are omitted.

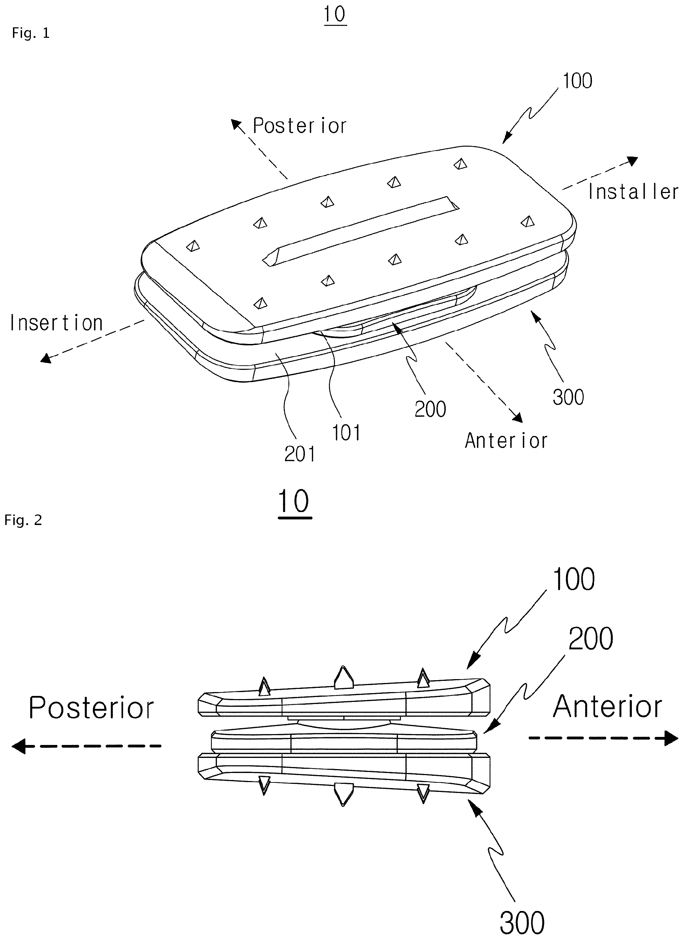

FIG. 1 illustrates an artificial disc 10 according to an embodiment of the present disclosure. First, directions are defined. A patient's abdominal direction is referred to as an anterior direction, and the patient's back direction is referred to as a posterior direction. Therefore, the anterior direction and the posterior direction are opposite to each other. In addition, a direction in which the artificial disc 10 is inserted during surgery is referred to as an insertion direction, and a direction, in which a device (not shown) for holding the artificial disc 10 is located, in the opposite direction to the insertion direction is referred to as an installer direction.

Also, an imaginary straight line determined in the anterior direction and the posterior direction is referred to as an anterior to posterior axis, and an imaginary straight line determined in the installer direction and the insertion direction is referred to as a lateral axis. Also, a height direction of the human body is referred to as a vertical axis. Therefore, the anterior and posterior axis, the lateral axis, and the vertical axis may form perpendicular to each other.

Next, if defining a plane, according to the definition in human anatomy, the plane is defined as a transverse plane with the vertical axis as the normal direction, a frontal plane that includes the vertical axis and has the anterior to posterior direction of the human body as the normal direction, and a sagittal plane that includes the vertical axis and has the left and right direction of the human body as the normal direction.

The artificial disc 10 includes an upper plate 100, an outer surface of which is basically in contact with an upper vertebrae to be inserted between two adjacent vertebrae, a lower plate 300, an outer surface of which is in contact with lower vertebrae, and an inserter 200 inserted between the upper plate 100 and the lower plate 300 so that the upper plate 100 rotates at a set angle with respect to the lower plate 300.

The upper plate 100, the inserter 200, and the lower plate 300 may include materials that are harmless to the human body. Because the upper plate 100 and the lower plate 300 are required to be fixed by contacting the vertebral body, a polymer material such as Poly-Ether-Ether-Ketone (PEEK), or a metal material such as titanium or a titanium alloy is preferable. In particular, titanium or titanium alloy is preferable because it has the characteristic of providing excellent osseointegration. In particular, so as to be advantageous for osteogenesis, osteoconduction, and osteoinduction, the roughness of a surface may be increased by sanding, deposition, coating, etc. to have a rough surface at a contact surface with the vertebral body, or it may be formed to include a porous structure through 3D printing.

Because the inserter 200 slides while contacting the upper plate 100 and the lower plate 300, a polymer material such as Ultra High Molecular Weight Polyethylene (UHMWPE) with excellent wear resistance or a metal material such as a cobalt chromium (Co-Cr) alloy may be used.

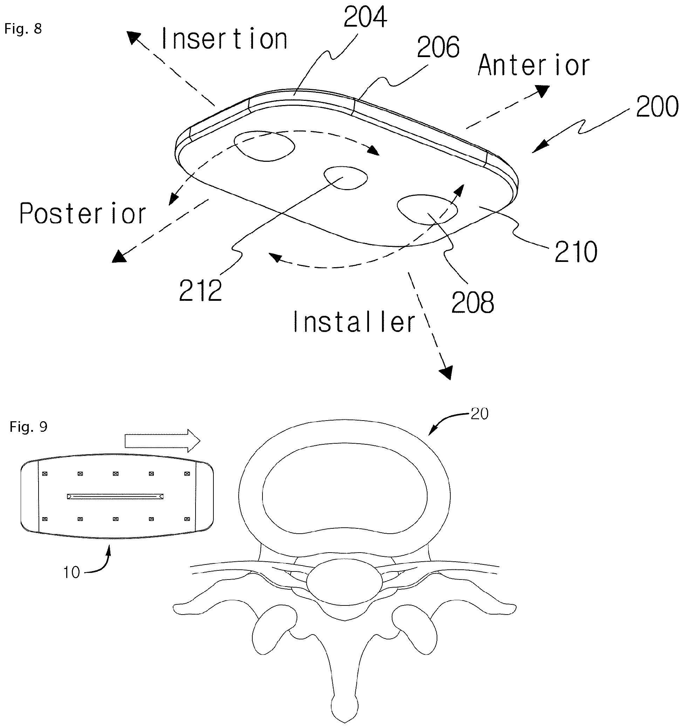

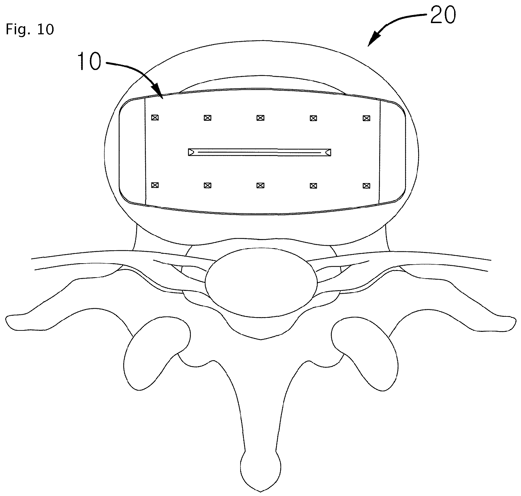

As shown in FIGS. 9 and 10, because the artificial disc 10 is inserted from a patient's lateral direction, the artificial disc 10 should be adjusted to a width along an anterior to posterior axis of the vertebra 20, which is narrower than a left-right length along the lateral axis of the vertebra 20. In addition, because a surgery is performed in a lateral direction, a minimal invasive surgery (MIS) is performed, which has a long and narrow surgical path. Therefore, the artificial disc 10 has an approximately rectangular shape in which a length in the lateral axis direction is long and a length in the anterior to posterior axis direction is narrow.

Therefore, an upper wing portion 101 and a lower wing portion 301 that extend along the lateral axis are formed in the upper plate 100 and the lower plate 300 while the inserter 200 is inserted between the upper plate 100 and the lower plate 300. That is, the upper plate 100 and the lower plate 300 extend lengthways to the outside of a width of the lateral axis direction of the inserter 200, and thus, the upper plate 100 and the lower plate 300 have an approximately rectangular shape in which a length in the lateral axis direction is long and a length in the anterior to posterior axis direction is narrow

As described below, the upper plate 100 and the lower plate 300 of the artificial disc 10 are bent left and right on a coronal plane with respect to the inserter 200 due to the shape of the inserter 200. At this time, if the inserter 200 has the same length as the upper plate 100 or the lower plate 300, the amount of movement of the upper plate 100 and the lower plate 300 is limited due to the thickness of the inserter 200 during left-right bending. Therefore, there is an advantage in that a size of the left-right bending of the artificial disc 10 may be increased by forming the upper wing portion 101 and the lower wing portion 301 and relatively reducing the length of the inserter 200 in the lateral axis direction.

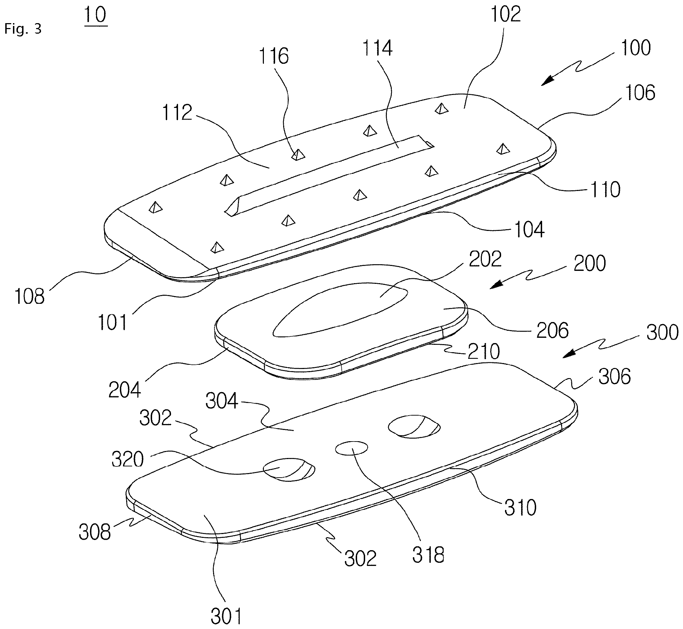

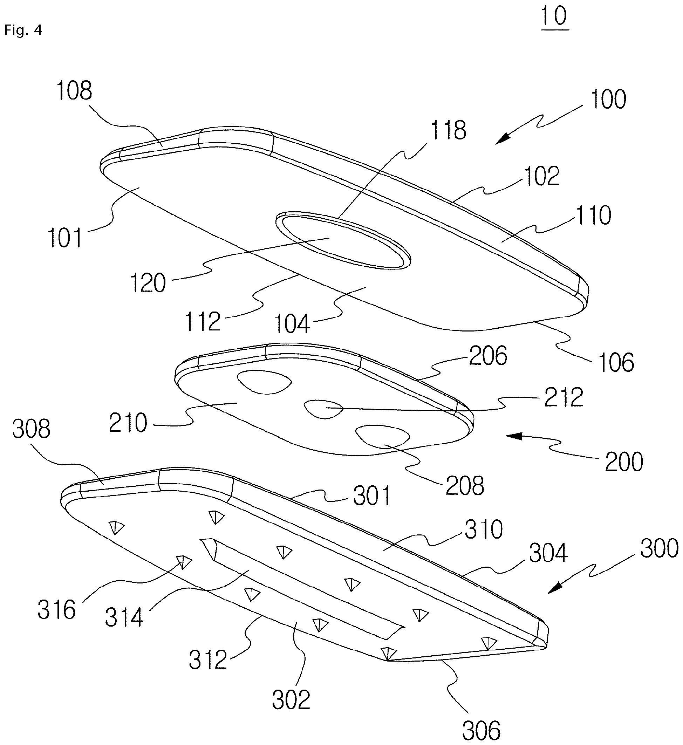

As shown in FIGS. 3 and 4, the upper plate 100 has an approximately hexahedron of an upper front wall portion 110 in a forward direction, an upper rear wall portion 112 in a posterior direction, an upper mechanism wall portion 106 in a mechanism direction, an upper insertion wall portion 108 in the insertion direction, an upper plate outer surface 102 that comes into contact with the vertebrae, and an upper plate inner surface 104 in the opposite direction to the upper plate outer surface 102, which is approximately a hexahedron.

Likewise, the lower plate 300 has an approximately hexahedron of a forward lower front wall portion 310, a backward lower rear wall portion 312, a mechanism-oriented lower mechanism wall portion 306, an insertion-oriented lower insertion wall portion 308, a lower plate outer surface 302 that comes into contact with the vertebrae, and a lower plate inner surface 304 opposite to the lower plate outer surface 302.

In the lumbar vertebrae of the human body, a lordosis angle of the disc increases from the disc closer to the thoracic vertebrae to the disc closer to the sacrum.

In the artificial disc 10, the shapes of the upper plate 100 and the lower plate 300 are formed so that the thickness in the forward direction is greater than the thickness in the backward direction in order to correspond to the lordosis angle of the disc. Therefore, as shown in FIG. 2, the upper plate outer surface 102 of the upper plate 100 and the lower plate outer surface 302 of the lower plate 300 may be formed to be inclined to each other.

In this case, the upper plate inner surface 104 of the upper plate 100 and the lower plate inner surface 304 of the lower plate 300 remain parallel to each other.

As a result, the artificial disc 10 allows the lordotic angle of the disc to be varied, not just 0 degrees, thereby allowing the lordotic angle of the patient's operated disc to have the proper value. Thus, artificial disc 10 may maintain an overall curvature of the lumbar spine.

The artificial disc 10 is inserted between the vertebral bodies from the patient's lateral direction as shown in FIGS. 9 and 10. When the artificial disc 10 is inserted, a strong impact is applied. In the artificial disc 10, in a state that the upper plate 100 and the lower plate 300 are fixed to the vertebral body 20, a contact movement between the inserter 200 and the upper plate 100 and a contact movement between the inserter 200 and the lower plate 300 should occur. Therefore, the upper plate 100 and the lower plate 300 need to be strongly fixed to the vertebral body 20.

Therefore, an upper keel portion 114 and a lower keel portion 314 may be formed along the lateral axis on the upper plate outer surface 102 of the upper plate 100 and the lower plate outer surface 302 of the lower plate 300, respectively. As a result, the upper keel portion 114 and the lower keel portion 314 forcefully form a groove on a surface of the vertebral body when the artificial disc 10 is inserted into the vertebral body 20 and come into close contact with the vertebral body. Therefore, not only does a surface area between the vertebral body and the upper plate 100 and the lower plate 300 increase, but also slipping may be prevented when a load is transmitted from the vertebral body to the upper plate 100 and the lower plate 300.

In addition, as shown in FIGS. 3 and 4, a plurality of upper protrusions 116 and a plurality of lower protrusions 316 may be formed on the upper plate outer surface 102 of the upper plate 100 and the lower plate outer surface 302 of the lower plate 300.

The upper protrusions 116 and lower protrusions 316 may have a quadrangular pyramid shape having a rectangular cross section composed of four line segments each perpendicular to the lateral axis and the anterior to posterior axis. As a result, the upper protrusions 116 and the lower protrusions 316 may support a load in a direction along the lateral axis and a direction along the anterior to posterior axis, thereby supporting the artificial disc 10 in various directions.

Next, the inserter 200 will be described.

In the human body, discs serve as ligaments that connect the vertebrae, act as the center of motion for the spine, and cushion the spine's shock. In particular, the spine movement by the disc is divided into three types: anterior to posterior flexion-extension motion, lateral bending, and axial rotation.

First, the anterior to posterior flexion-extension motion is a motion performed in the sagittal plane and is a motion in which the body is bent forward or backward. The lateral bending is a motion performed in the coronal plane and is a motion in which the body is tilted left and right. The axial rotating is a motion called a twist motion, and is a motion in which the torso is twisted and rotated while maintaining a straight state.

The main feature of the inserter 200 is dividing and distributing the three motions above. That is, an upper dome portion 202 arranged on an upper side of the inserter 200 is in charge of anterior to posterior flexion-extension and lateral bending, and the first axial rotation portion arranged on a lower side of the inserter 200 is formed to be in charge of axial rotation. The upper dome portion 202 is in sliding contact with an upper groove 120 formed in the upper plate 100, and a first axial rotation portion is in sliding contact with a second axial rotation portion formed in the lower plate 300.

To this end, the upper dome portion 202 is formed on the upper side of the inserter 200, and the first axial rotation portion is formed on the lower side of the inserter 200.

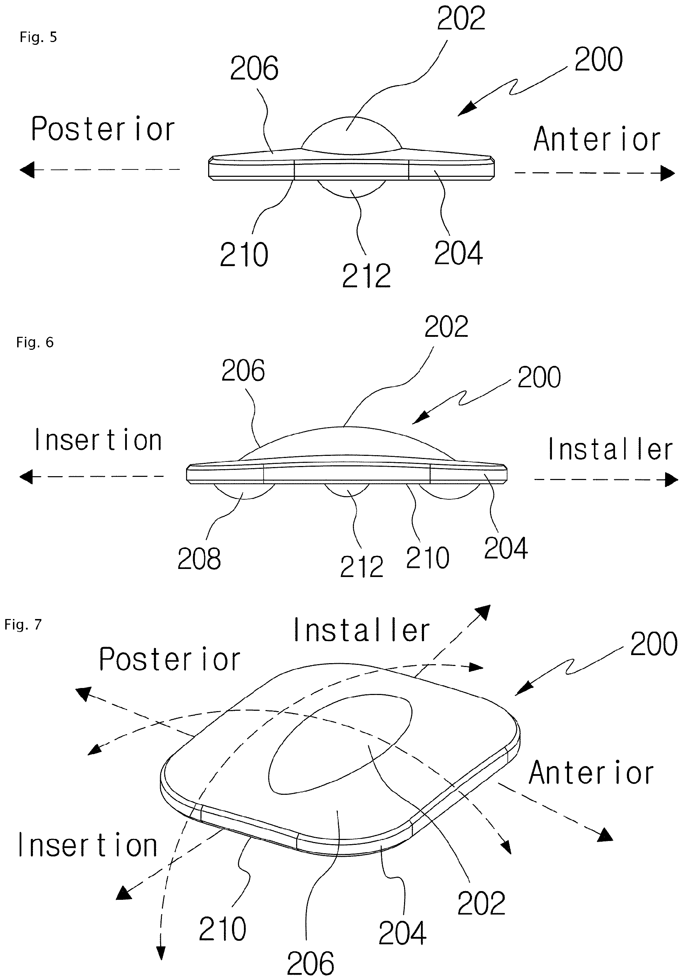

As shown in FIGS. 5 to 7, the upper dome portion 202 is formed in an oval hemispherical shape in which the vertical axis is arranged on the lateral axis line and a short axis is arranged on the anterior to posterior axis line.

First, referring to FIG. 5, the upper dome portion 202 is formed to form a front-back curve along the anterior to posterior axis line. In addition, referring to FIG. 6, the upper dome portion 202 is formed to form a left-right curve along the lateral axis line.

Therefore, by combining the two curved surfaces, the upper dome portion 202 forms an elliptical hemispherical shape as shown in FIG. 7.

Also, as shown in FIG. 3, in the upper plate 100, the upper groove 120 is formed to correspond to the upper dome portion 202 to make sliding contact with the upper dome portion 202. That is, the upper groove 120 also simultaneously has a curve corresponding to a curve formed forward and backward along the anterior to posterior axis of the upper dome portion 202 and a curve corresponding to the curve formed left and right along the lateral axis of the upper dome portion 202. In particular, a maximum depth of the upper groove 120 is formed less than a maximum height of the upper dome portion 202, and thus, the upper groove 120 may only contact a unit of the upper dome portion 202.

An inserter body 204 is formed around a lower side of the upper dome portion 202. The inserter body 204 is widely spread like a flange, and in the embodiment of the present disclosure, it is formed in an approximately square or rectangular shape but may also be formed in an oval shape.

An inserter lower surface 210 of the inserter body 204 is formed flat to make sliding contact with the lower plate inner surface 304 of the lower plate 300. Because the lower plate inner surface 304 makes sliding contact, a load applied to the upper plate 100 may be stably transmitted to the lower plate 300 through the inserter 200.

In addition, the inserter lower surface 210 of the inserter body 204 is formed so that, while in a flat state, the inserter upper surface 206 has a thickness gradually reduced from the upper dome portion 202 to an end of the inserter body 204. Looking at the insert body 204 in FIG. 5 and FIG. 6, it may be confirmed that the thickness of the inserter upper surface 206 is gradually reduced along the lateral axis and the anterior to posterior axis.

An upper stopper 118 is formed around the upper groove 120. The upper stopper 118 is formed to protrude from the upper plate inner surface 104.

The angular momentum of the upper plate 100 with respect to the lower plate 300 is limited by the upper stopper 118 coming into contact with the inserter upper surface 206 of the inserter body 204.

In particular, by forming the inserter upper surface 206 to have a thickness gradually reduced from the upper dome portion 202 to the end of the inserter body 204, the angular momentum of the upper plate 100 with respect to the lower plate 300 may be increased. That is, compared to the inserter upper surface 206 having a constant thickness, the gradual reduction of the thickness of the inserter upper surface 206 toward the end of the inserter body 204 may further increases an inclination angle formed by the upper plate 100 and the lower plate 300 when the upper stopper 118 touches the inserter upper surface 206.

According to an embodiment of the present disclosure, as shown in FIG. 4, the first axial rotation portion arranged on the lower side of the inserter 200 is a lower outer protrusion 208 formed on the inserter lower surface 210 of the inserter body 204, and the second axial rotation portion is a lower outer groove 320 formed on the lower plate 300, and the lower outer groove 320 is formed in an arc shape.

The lower outer protrusion 208 is arranged symmetrically with respect to the center of rotation along the vertical axis of the inserter 200. If there are more than three lower outer protrusions 208, they may be arranged at a certain angle with respect to the center of rotation along the vertical axis.

In an embodiment of the present disclosure, the lower outer protrusion 208 is formed in an approximately hemispherical shape, the lower outer groove 320 is formed in an arc shape so that the lower outer protrusion 208 moves within a certain angular range, and in particular, an end of the lower outer groove 320 has a shape in which the lower outer protrusion 208 may be seated.

As another example of the first axial rotation portion, it is also possible that an arc-shaped groove or hole may be formed in the inserter body 204, and a protrusion may be formed on the lower plate inner surface 304 of the lower plate 300.

In addition, a lower center protrusion 212 may be formed at the center of rotation along the vertical axis of the inserter lower surface 210 of the inserter 200, and a lower center groove 318 corresponding to the lower center protrusion 212 may be formed on the lower plate 300. Conversely, a groove may be formed at the center of rotation along the vertical axis of the inserter lower surface 210, and a protrusion corresponding to the groove may be formed on the lower plate 300.

By combining the lower center protrusion 212 and the lower center groove 318, not only the inserter 200 may be stably rotated relative to the lower plate 300, but also there is an advantage in that a load applied with respect to the inserter 200 in the lateral axis or the anterior to posterior axis direction or in any direction in a transverse section may be stably supported.

The structure of the artificial disc 10 according to the present disclosure is as described above. Next, the movement of the artificial disc 10 will be described in detail.

The artificial disc 10 may perform lateral bending and anterior to posterior flexion-extension motion of the upper plate 100 relative to the lower plate 300 by moving through contact between the upper dome 202 of the inserter 200 and the upper groove 120 of the upper plate 100.

That is, as illustrated in FIG. 7, the upper plate 100 undergoes lateral bending in the frontal plane relative to the lower plate when the upper wing 101 of the upper plate 100 approaches the lower wing 301 of the lower plate 300 in the installer direction while the upper groove 120 is in contact with the upper dome 202, or when the upper wing 101 of the upper plate 100 approaches the lower wing 301 of the lower plate 300 in the installer direction while the upper groove 120 is in contact with the upper dome 202.

In addition, the upper plate 100 undergoes an anterior to posterior flexion-extension motion in the sagittal plane with respect to the lower plate when the upper front wall portion 110 of the upper plate 100 approaches the lower front wall portion 310 of the lower plate 300 in the anterior direction while the upper groove 120 is in contact with the upper dome 202, or when the upper rear wall portion 112 of the upper plate 100 approaches the lower rear wall portion 312 of the lower plate 300 in the posterior direction while the upper groove 120 is in contact with the upper dome 202 while moving backward.

At this time, the upper stopper 118 around the upper groove 120 comes into contact with the inserter upper surface 206 of the inserter body 204, thereby limiting the amount of lateral bending and anterior to posterior flexion-extension motion.

In addition, as shown in FIG. 8, when the lower center protrusion 212 of the inserter 200 is inserted into the lower center groove 318 of the lower plate 300, the axial rotation of the upper plate 100 in a transverse plane with respect to the lower plate 300 may be achieved by the movement of the lower outer protrusion 208 of the inserter 200 along the lower outer groove 320 of the lower plate 300.

At this time, the amount of movement of the axial rotation is limited when the lower outer protrusion 208 contacts both ends of the lower outer groove 320,

When such anterior to posterior flexion-extension motion, lateral bending, and axial rotation occur in combination, the artificial disc 10 may move similarly to the movement of a human disc.

According to the artificial disc 10 according to the present disclosure, an artificial disc replacement (ADR) is possible by inserting the artificial disc 10 from lateral approach, and because a surgery is performed by inserting the artificial disc 10 from the lateral approach, an anterior longitudinal ligament and a posterior longitudinal ligament (PLT), which stabilize the spine during the surgery, may be preserved, and thus, the force transmitted to the posterior joint may be reduced, thereby maintaining the spine more stably.

In addition, the anterior longitudinal ligament is a ligament that plays the most important role in preventing excessive bending of the spine and maintaining the stability of the joint between the vertebral bodies. Therefore, if the anterior longitudinal ligament is cut, the spine rotates beyond the original rotation range due to the absence of the anterior longitudinal ligament, and as a result, strain is put on the posterior joint. The present disclosure may solve the problems of conventional anterior artificial disc insertion surgery by preserving the anterior longitudinal ligament.

In addition, according to the artificial disc 10, because motion control is possible while both the anterior longitudinal ligament and the posterior longitudinal ligament are preserved, not only may the burden on the posterior joint be reduced, but also the motion may be optimized because the motion is controlled with the help of the anterior longitudinal ligament and the posterior longitudinal ligament.

As described above, although the present disclosure has been described with reference to preferred embodiments thereof, it will be understood by those skilled in the art that the present disclosure may be variously modified and changed within the spirit and scope of the present disclosure as set forth in the claims below.

### Industrial Applicability

In the case of an anteriorly inserted artificial disc, assistance from a thoracic surgeon or a vascular surgeon is required to secure an incision and surgical route. However, in the case of a laterally inserted spinal implant, because there is no interference from organs from skin to the spine, there is an advantage in that surgery may be performed only by an orthopedic surgeon or a neurosurgeon.

Therefore, the artificial disc according to the present disclosure is expected to be able to replace not only existing artificial discs but also laterally inserted fusion cages because the artificial disc overcomes the problems and disadvantages of conventional anteriorly inserted artificial discs and allows the patient to maintain mobility while being inserted laterally.

## Claims

1. An artificial disc for insertion between two adjacent vertebrae, the artificial disc comprising:
an upper plate contacting an upper vertebra;
a lower plate contacting a lower vertebra; and
an inserter inserted between the upper plate and the lower plate to allow the upper plate to rotate by a set angle with respect to the lower plate,
wherein the upper plate and the lower plate are **characterized in that** an upper wing portion and a lower wing portion are formed along a lateral axis while the inserter is inserted.

2. The artificial disc of claim 1, wherein
the upper plate and the lower plate each have a thickness greater in an anterior direction than in a posterior direction.

3. The artificial disc of claims either 1 or 2, wherein
an upper keel portion and a lower keel portion are respectively formed along a lateral axis on an upper plate outer surface of the upper plate and a lower plate outer surface of the lower plate.

4. The artificial disc of claims either 1 or 2, wherein
a plurality of upper protrusions and lower protrusions are respectively formed on an upper plate outer surface of the upper plate and a lower plate outer surface of the lower plate.

5. The artificial disc of claim 4, wherein
the plurality of upper protrusions and the lower protrusions each have a quadrangular pyramid shape with a quadrangular cross section including four line segments each perpendicular to the lateral axis and the anterior to posterior axis.

6. The artificial disc of claims either 1 or 2, wherein
the inserter includes an upper dome portion on an upper side thereof and a first axial rotation portion on a lower side thereof,
an upper groove corresponding to the upper dome portion is formed in the upper plate for a sliding contact with the upper dome, and
a second axial rotation portion is formed on the lower plate that contacts the first axial rotation portion.

7. The artificial disc of claim 6, wherein
the upper dome portion is formed in an elliptical hemispherical shape in which a lateral axis is on the lateral axis line and minor axis is on the anterior to posterior axis line, and the upper plate performs lateral bending and an anterior to posterior flexion-extension motion with respect to the lower plate by moving the upper dome portion and upper groove contacting each other.

8. The artificial disc of claim 6, wherein
a maximum depth of the upper groove is less than a maximum height of the upper dome portion.

9. The artificial disc of claim 6, wherein
an upper stopper is formed around the upper groove, and an angular momentum of the upper plate is limited by the upper stopper coming into contact with an inserter upper surface of the inserter body formed around the upper dome portion.

10. The artificial disc of claim 9, wherein
an inserter lower surface of the inserter body is flat, and the inserter upper surface is formed so that a thickness thereof is gradually reduced from the upper dome portion to an end of the inserter body.

11. The artificial disc of claim 6, wherein
the first axial rotation portion is a lower outer protrusion formed on the inserter lower surface, the second axial rotation portion is a lower outer groove formed on the lower plate, and the above lower outer groove is formed in an arc shape.

12. The artificial disc of claim 11, wherein
a lower center protrusion is formed at a center of the inserter lower surface, and a lower center groove corresponding to the lower center protrusion is formed on the lower plate.
